# EUROPEAN PATENT SPECIFICATION

(11) **EP 3 730 096 B1**
(45) Date of publication and mention of the grant of the patent: **25.09.2024**
(21) Application number: 18892879.0
(22) Date of filing: 20.12.2018
(51) Int. Cl.: A61F 2/28, A61L 27/02, A61C 8/00, A61F 2/30

(54) **BONE IMPLANT**
KNOCHENIMPLANTAT
IMPLANT OSSEUX

(30) Priority: 20.12.2017 RU 2017144895
(43) Date of publication of application: 28.10.2020
(73) Proprietor: Pugach, Andrey, Moscow 115054 (RU)
(72) Inventor: Pugach, Andrey, Moscow 115054 (RU)
(74) Representative: Jeck, Anton
(86) International application number: PCT/RU2018/050165
(87) International publication number: WO 2019/125225

(56) References cited:
- KR-B1- 100 698 507
- US-A1- 2008 262 612
- SYCH E E ET AL: "Structure and Properties of Permeable Highly Porous Glass-Ceramics for Orthopedics and Traumatic Surgery", POWDER METALLURGY AND METAL CERAMICS, SPRINGER NEW YORK LLC, UNITED STATES, UKRAINE, vol. 55, no. 5, 6 October 2016 (2016-10-06), pages 319 - 327, XP036075016, ISSN: 1068-1302, [retrieved on 20161006], DOI: 10.1007/S11106-016-9808-X
- P.K. TOMASZEWSKI ET AL: "Simulated bone remodeling around two types of osseointegrated implants for direct fixation of upper-leg prostheses", JOURNAL OF THE MECHANICAL BEHAVIOR OF BIOMEDICAL MATERIALS, vol. 15, 2 August 2012 (2012-08-02), AMSTERDAM, NL, pages 167 - 175, XP055619967, ISSN: 1751-6161, DOI: 10.1016/j.jmbbm.2012.06.015
- SYCH E.E. ET AL.: "Structure and properties of permeable highly porous glass-ceramics for orthopedics and traumatic surgery", POWDER METALLURGY AND METAL CERAMICS, vol. 55, no. 5-6, 2016, pages 319 - 327, XP036075016, doi:10.1007/s11106-016-9808-x
- TOMASZEWSKI P.K. ET AL.: "Simulated bone remodeling around two types of osseointegrated implants for direct fixation of upper-leg prostheses", JOURNAL OF THE MECHANICAL BEHAVIOR OF BIOMEDICAL MATERIALS, vol. 15, 2 August 2012 (2012-08-02) - November 2012 (2012-11-01), pages 167 - 175, XP055619967
- FAVERANI LEONARDO PEREZ ET AL.: "Surgical techniques for maxillary bone grafting - literature review", REVISTA DO COLÉGIO BRASILEIRO DE CIRURGIÕES, vol. 41, no. 1, January 2014 (2014-01-01) - February 2014 (2014-02-01), pages 61 - 67, XP055619971

## Description

The invention relates to the field of medicine and medical technology, namely to bone implants. These are the implants that replace or supplement bone tissue or connect parts of the bones, typically they are fully or partially fixed in the bone tissue. The closest prior art is document US 4171544, which defines the preamble of claim 1.

Implants of inorganic materials that are most commonly used as bone implants can be divided into two major groups - metallic implants and implants made of oxide materials, in particular aluminum oxide, zirconium oxide, silicon oxide.

Metallic implants are widely used after the discovery of osseointegration of titanium (Branemark, 1969). Pure titanium and its alloys (Ti with V, Al, Nb, Zr) are still used most frequently as a basis for implants. To improve biocompatibility, the surface of titanium implants is often modified. Sandblasting, etching and oxidation increase the microroughness and hydrophilicity of the surface of the titanium implant and lead to an increase in the speed of osseointegration.

The advantage of implants made of oxide-based materials is chemical resistance. Polycrystalline, in particular ceramic, as well as monocrystalline and vitreous oxide materials are used for the manufacture of implants. There is known an implant made of ceramics containing polycrystalline alumina (Hemke, 1975). Also known is a monocrystalline alumina implant (Hirabayashi, 1978). The compressive and tensile strength and the chemical resistance of the single-crystal alumina are very high.

Implants made from ZrO2-based oxide ceramics have high strength and high aesthetic quality; they are second in frequency of application after titanium implants. Yttrium-stabilized zirconia-based materials have high chemical and biological stability, high strength, relatively low Young's modulus (Yilmaz, 2007).

Silicon oxide based bone implants have been known since the creation of 45S5 bioglass with the composition of SiO2-P2O5-CaO-Na2O (Hench, 1971). Bioglass can be used in powder form as a filler for bone grafting in filling bone defects. Osseointegrated bioglass is later replaced with live bone tissue. It was originally assumed that osseointegration is induced by P2O5 and CaO from 45S5 bioglass that approximate the composition of the bioglass to the hydroxyapatite composition of the bone tissue. However, it has been further found that both P2O5-free and CaO-free silicate glasses do also have osseoinductive properties (Hench, 1979).

Increased strength and reduced resorption of silicate materials is also known for artificial teeth made of lithium disilicate glass ceramics (Barrett, 1980) and apatite wollastonite glass ceramics (Yoshida, 1985). Silicate bioceramics of such compositions have excellent compatibility with the surrounding tissues and high strength, much higher than that of bioglass.

Biocompatibility and osseointegration ability of silica-containing implants, similar to titanium, depends on the surface structure. Experiments to determine the formation of hydroxyapatite crystals on various surfaces of silicon oxide materials were performed (Li, 1992) by placing samples in a simulated body fluid (SBF). SBF test is often used to evaluate the possibility of osseointegration and as an indicator of the biological compatibility of the materials. It has been found that the hydroxyapatite layer is formed in SBF on the surface of the silica gel with a developed rough surface, but this is not the case for silica glass with a smooth surface.

### Summary of Invention

The present invention is defined in claim 1 and concerns a bone implant that is completely or partially made of silicate rock or natural or artificial mineral aggregates with a dense tangled-fibrous or fine-crystalline structure known under the general name "jade".

### Technical problems

The most important properties of bone implants influencing the success of their application are mechanical strength, chemical resistance, biocompatibility, low electrochemical activity, Young's modulus, the ability to integrate into the bone (osseointegration).

Titanium implants have excellent strength, osseointegration ability, established medical protocols and long-term use. The disadvantage of metallic implants, including titanium implants is the electrochemical activity of metals that can cause different complications in the use of implants of titanium and its alloys (Pozhitkov, 2015).

Coating the surface of titanium implants with hydroxyapatite or bioglass may improve osteoplastic properties of the implant and reduce the electrochemical activity, but this effect is short-lived and poor due to resorption of the applied surface layer.

The alumina is very durable, but biologically inert, has a high Young's modulus and a positive zeta potential at physiological pH. This can lead to difficulties in osseointegration and bone resorption over time with prolonged use of the implant.

Possible ageing of zirconia ceramics in an aqueous environment makes an unreliable prognosis of the stability of zirconia implants during long-term use.

Silicate bioglass have high osseoinductive and osteoconductive properties, but have low strength, are fragile and susceptible to resorption with a long stay in the bone. These properties make it difficult to use them as a non-resorbable bone implants. Lithium disilicate and apatite-wollastonite silicate bioceramics have higher strength than that of biglasses, but have unknown prognosis of prolonged use.

These data show the promise of the use of bone implants from silicate materials in medicine, but the properties of the implants from many of such materials limit the scope of their application.

### Solution to Problem

In the present invention, to expand the range of medical devices for implantation into bone tissue and to obtain high-strength implants capable of osseointegration, bone implants are made from silicate mineral aggregates and rocks with dense tangled-fibrous or fine-crystalline structure, known under the general name "jade".

As jade are known, among others, the following mineral aggregares and rocks: jadeite jade Na(AI,Fe)Si2O6, nephrite jade Ca2(Mg,Fe)5Si8O22(OH)2, xonotlite (xonothlite jade) Ca6(Si6O17(OH)2), vesuvianite (Californian jade) Ca10(Mg,Fe)2Al4((OH)4(SiO4)5(Si2O7)2), bowenite (new jade) (Mg,Fe)3(OH)4Si2O5, pectolite (Alaskan jade) NaCa2(Si3O8(OH)), hydrogrossular (Transvaal jade) Ca3Al2(SiO4)2(OH)4.

Like many minerals, mineral aggregates and rocks, jades may have slightly variable compositions depending on the formation conditions and deposit locations due to the phenomenon of isomorphism - the variability of the composition while maintaining the crystalline structure and material properties. Jades are massive and tough mineral aggregates and silicate rocks, occurring in nature, or synthetic materials of similar structure and composition. As it turned out unexpectedly, implants of these materials have the optimum characteristics for integration into the bone tissue.

These mineral aggregates and rocks are resistant to cracking, have dense tough structure, which provides high strength in combination with a relatively low modulus of elasticity. With the exception of jadeite jade, other jades are hydrosilicates. They are semi-transparent, translucent in thin layers and usually acquire a characteristic roughened matt surface. Since ancient times, jades were highly appreciated in the stone-cutting industry, a lot of tools (knives, axes) and art objects (cameos, statuettes, jewelry) were made of jade. The mechanical properties of these mineral aggregates can compete with titanium and steel, and compressive strength is superior to many alloys. However, the properties of jades related to the possibility of integration into the bone tissue, did not attract the attention of researchers.

The composition of jades includes silicon oxide, which makes them promising in terms of biological compatibility and ability to osseointegrate. The fibrous structure of these silicates provides elasticity and microroughness required to form a strong bond with bone tissue. Jades as dielectrics are not involved in the electrochemical reactions, are hydrophilic, have a negative zeta potential at physiological conditions. Consideration of the set of jades properties allow us to hope for high quality of bone implants made of jades according to the present invention.

When evaluating the application possibilities of jades as a material for bone implants their high strength has been confirmed experimentally. Polished jade samples without cracks, inclusions and other defects were used in tests. It was confirmed that jades have a high compressive strength (more than 100MPa), bending strength (more than 50 MPa), high impact strength, high fracture toughness (greater than 2 MPa/ m1/2). Young's modulus of jades is relatively low and is comparable with the modulus of elasticity of titanium (100-250 GPa).

Test of the properties of materials in aqueous solutions showed that jades are hydrophilic with wetting angle being less than 90 degrees. The zeta potential is negative in jades, which is typical for materials capable of osseointegration.

These results show the promises of using jades for the production of bone implants. However, the interaction of these silicates with bone tissues has not been studied previously. The following results showed that implants made of jades have unexpectedly good biological compatibility and ability to osseointegrate.

For initial evaluation of the biocompatibility jades were placed in SBF-solution according to the procedure (Li, 1992). After 1-4 weeks of the start of the experiment, hydroxyapatite crystals were formed on the surface of jades thus confirming the perspective of biological compatibility and the possibility of osseointegration of jade implants according to the invention.

To test the biocompatibility of jade implants in vitro, their interaction with mouse osteoblast cells was studied. It has been found that jade implants do not have a toxic effect on cells, osteoblasts attach and proliferate on the surface of jades.

Implantation of jade implants into the bone tissue was performed to verify the osseointegration. Cylindrical implants made of jades were placed in a hole in the tibia of guinea pigs. At the ends of the implants there were facets used to measure the torque. After 6 weeks of experiment the torque required for breakdown of implants was measured, and it was found that torque after engraftment of the implant exceeds the torque right after the inoculation of the implant into the bone tissue (more than 5 N/cm). These data confirm the ability of osseointegration of implants according to the present invention.

### Advantageous Effects of Invention

Bone implants made of jades according to the present invention have high mechanical properties, high hydrophilicity, high biological compatibility, ability to osseointegrate, low electrochemical activity, high aesthetic characteristics. An additional advantage of jade implants is their dielectric properties. Low conductivity reduces problems with MRI, CT and other medical procedures that use electrical signals. The properties inherent to jade implants show the promises of their use in surgery, orthopedics, dentistry.

### Description of Embodiments

An exemplary and non-exhaustive list of jade implants according to the present invention comprises implants for replacement of bone tissue, volumetric bulk fillers in granular form for inclusion in the composition for filling bone tissue defects, bone screws, implants for bone fusion, fixing pins implants or pins for implants, implants for cranial reconstruction, orthopedic implants, transdermal osseointegrated implants, dental implants.

For orthopedic implants, the high strength of jade is of particular importance, which allows their use in implants in joints and heavy duty parts of endoprostheses. The group of orthopedic implants includes implants of hip joint, implants of knee joint, implants of shoulder joint, implants of elbow joint, implants of wrist joint, implants of ankle joint, implants of subtalar joint, implants of metatarsal joint, implants of joints of fingers, or in general, implants of joints, implants of spinal column, including implants of vertebra and implants of intervertebral disk, implants of head of radial bone, thumb implants, implants for osteotomy (high tibia osteotomy). Particularly preferred orthopedic implants are implants of joints, in particular, implant of hip joint and implant of knee joint. Implant of hip joint may be prosthesis of head and prosthesis of diaphysis (stem of endoprosthesis or diaphysis of femur) and cotyloid cavity prosthesis. The knee joint implant can include tibial and femoral components of the knee joint.

The next group of jade implants according to the present invention are transcutaneous osseointegrated implants (endoexoprostheses) which are integrated in the bone of the distal part of the joint on the one side, extra-bone part of the implant extends beyond the body through the tissues and the patient's skin, and the external part of the implant is used for fixation of external exoprotheses. For endoprostheses as well as for orthopedic implants the high strength of jade implants is highly valuable. A significant advantage for the endoexoprostheses made of jade can be the low electrochomic activity and the insulating properties of such implants. Group of percutaneous osteointegrated implants includes endoprothesis for legs, endoexoprostheses for hands, endoexoprostheses for fingers, endoexoprostheses for cosmetic surgery.

The group of dental implants includes the dental implant itself (spiral, cylindrical, conical or lamellar shape), dental abutment, dental implant with integrated abutment, dental crown, dental implant with integrated abutment and crown, dental beam, dental insert, dental pin.

Structure and color of implant body are extremely important for "smile zone" dental implants. It should be noted that the color of jade implants according to the invention may vary significantly depending on the composition of jade, its formation conditions, its source. Classic jade usually has green tones but it can be almost any color. White jades are also common, and is often valued more than green. Natural white jade, which has the color of "mutton fat", is well known and has a high cost in some countries. It is widely used in the production of high-end jewelry. In nature there exist also white jadeite, xonothlite, vesuvianite, bowenite and pectolite jades. Synthetic jades can also be of any color, including white.

Jade dental implant may be helical, cylindrical, conical, or plate-like shape, demountable or combined with an abutment. In the detachable implant the anchoring of the dental abutment to the dental implant root is often carried out by means of an internal screw. The dielectric and mechanical properties of jade implant can provide electrochemical isolation of the internal fastening screw from biological fluids of the body. This insulation allows the use of screws of different materials, not only titanium, but also made from steel or other alloys for fastening parts of demountable dental implant. Other dental jade products such as dental crown, dental insert, dental pin can also get the benefits of osseointegration with the bone tissue of the tooth or jaw. Dental root implant, abutment and the crown can also be made of white jade to match to the patient's teeth tone. The consumer characteristics of white jade implants for the smile area, in particular cosmetic, exceed the consumer characteristics of metallic implants.

A preferred dental implant is an osseointegratable jade beam properly adjusted for attachment to the alveolar bone. The shape and the size of the beam may be chosen individually for the patient, the alveolar bone may be prepared for the area of contact of the bone tissue with the beam, shape adjustments of the bone may be made corresponding to the shape of the bottom of the beam. The beam framework, which have holes for fastening, can be fastened to the bone through the holes provided with the prepared set of one or more endosseous implants or bone screws. Osseointegration of the beam leads to complex subperiosteal or endosseous-subperiosteal implant combining integrated beam and helical implant or a bone screw. Thus a combined implant is securely fixed on to the alveolar bone and bone grafting can be minimized. This implant may be further used as a base for fixing dental prostheses, chewing evenly distributes the load on the area of contact of beam with jaw bone. The use of such structures is justified for the case of implantation in the molar region, or in the case of bone type 3 or 4, or for the case of an insufficient volume of alveolar bone for endosseous implantation.

A particularly preferred dental implant is dental implant with integrated abutment which can be applied in one-step protocol implantation. The crown may be fixed immediately after implantation or after the successful healing of the implant. The use of an implant with an integrated abutment increases the convenience for the doctor and the reliability of the prosthesis for the patient. A further simplification of the implant design is a one-piece artificial tooth made of white jade, where the endosseous implant, abutment and dental crown are combined into a single medical device. Such an implant can be a standard size product, can be further adjusted locally or made individually for a particular patient.

### Examples

EXAMPLE 1. Granules of filler for osteoplasty manufactured from pectolyte jade, also known as Alaska jade. The granules are sterilized, mixed with autologous bone chips, implanted as a filler into the bone tissue defect according to the agreed protocol.

Example 2. The intervertebral disc implant is made of vesuvianite, also known as California jade. It is manufactured according to the required shape and size, sterilized, implanted according to the agreed protocol.

EXAMPLE 3. Implant for cranial reconstruction manufactured from bowenite, also known as new jade. It is manufactured according to required shape and size using CAD/CAM technology, sterilized, implanted according to the agreed protocol.

Example 4: Endoprosthesis of cotyloid cavity of hip joint manufactured from nephrite jade. It is manufactured according to the required shape and size, sterilized, implanted according to agreed protocol.

Example 5. Integral head and stem of hip joint endoprosthesis manufactured from jadeite jade. It is manufactured according to the required shape and size, sterilized, implanted according to the agreed protocol.

Example 6. Femoral component of knee joint endoprosthesis manufactured from xonothlite jade. It is manufactured according to the required shape and size, sterilized, implanted according to the agreed protocol.

Example 7. Helical subgingival endoosseous dental implant manufactured from hydroglossular, also known as transvaal jade. It is manufactured according to the required shape and size, sterilized, implanted according to agreed protocol. Implant is used as an artificial tooth root for implantation into the alveolar process according to 2-step protocol.

Example 8. Dental screw endoosseous implant with an integrated abutment manufactured from white nephrite. It is manufactured according to the required shape and size, sterilized, implanted into the smile zone according to 1-step protocal.

Example 9. Single-block artificial tooth, in which the endoosseous screw implant, abutment, and tooth crown are combined.

It is manufactured according to the required shape and size from white nephrite, sterilized, implanted into the smile zone according to 1-step protocol. The crown is adjusted in place.

Example 10. An osseointegratable beam of white xonothlite.

It is manufactured according to the required shape and size using CAD/CAM technology. The beam is fixed to the alveolar bone through holes in the beam using a xonothlite screw implant. The beam is fixed to a groove in the bone tissue, which has been prepared according to the shape of the lower part of the beam. The beam compensates for the lack of bone tissue in the smile zone, serves as the basis for dentures.

Bone implants made of jade, can be applied in the areas of medicine, like surgery, orthopedics, traumatology, stomatology.

### Citation List

### Patent Literature

Heimke 1975 US Patent 3919723
Hirabayashi 1978 US Patent 4122605
Hench 1979 US Patent 4171544
Barrett 1980 US Patent 4189325
Yoshida 1985 US Patent 4560666

### Non Patent Literature

Branemark 1969 Scand. J. Plast. Reconstr. Surg., 3, 81-100
Hench 1971 J. Biomed. Mater. Res. Symposium No. 2 (Part 1), 117-141
Li 1992 J. Am. Ceram. Soc., 75 (S) 2094-2097
Yilmaz 2007 J. Prosth. Dent., 98, 120-128
Pozhitkov 2015 Plos One, 10 (10): e0140393

## Claims

1. A bone implant configured for osteointegration, **characterised in that** the bone implant comprises a jade material selected from a group consisting of a jadeite jade, nephrite jade, xonothlite jade, vezuvianite jade, pectolite jade, bowenite jade and hydrogrossular jade, wherein the jade material is a silicate mineral aggregate or rock with tangled-fibrous or fine- crystalline structure.

2. Bone implant according to claim 1, wherein the bone implant is selected from a group comprising implants for replacement of bone tissue, volume fillers in granular form for filling of bone tissue defects, wedge-shaped bone implants, bone screws, implants for bone fusion, fixing pin implants or fixing pins for implants, implants for cranial reconstruction, hip joint implants, knee joint implants, brachial joint implants, elbow joint implants, wrist joint implants, ankle joint implants, subtalar joint implants, metatarsal joint implants, finger joint implants, joint implants, spinal implants, implants of radial bone, thumb implants and implants for osteotomy

3. Bone implant according to claim 1, wherein the bone implant is selected from a group consisting of osseointegratable transcutaneous implants comprising endo exo prosthesis of leg, endo exo prosthesis of hand, endo exo prosthesis of finger and endo exo prosthesis for cosmetic surgery.

4. The bone implant of claim 1, wherein the bone implant is selected from a group of dental articles comprising dental implants, dental abutments, dental implants with integrated abutment, dental crowns, dental implants with integrated abutment and crown, dental beams, dental inserts and dental pins.

## Patentansprüche

1. Knochenimplantat, das für die Osteointegration konfiguriert ist, **dadurch gekennzeichnet, dass** das Knochenimplantat ein Jade-Material umfasst, das aus einer Gruppe ausgewählt ist, die aus Jadeit-Jade, Nephrit-Jade, Xonothlit-Jade, Vezuvianit-Jade, Pektolit-Jade, Bowenit-Jade und hydrogrosser Jade besteht, wobei das Jade-Material ein Silikat-Mineral-Aggregat oder -Gestein mit verworren-faseriger oder feinkristalliner Struktur ist.

2. Knochenimplantat nach Anspruch 1, wobei das Knochenimplantat ausgewählt ist aus der Gruppe bestehend aus Implantaten zum Ersatz von Knochengewebe, Volumenfüllern in Granulatform zum Auffüllen von Knochengewebedefekten, keilförmigen Knochenimplantaten, Knochenschrauben, Implantaten zur Knochenfusion, Fixierstiftimplantaten oder Fixierstiften für Implantate, Implantate für die Schädelrekonstruktion, Hüftgelenksimplantate, Kniegelenksimplantate, Brachialgelenksimplantate, Ellenbogengelenksimplantate, Handgelenksimplantate, Sprunggelenksimplantate, Senkfußgelenksimplantate, Mittelfußgelenksimplantate, Fingergelenksimplantate, Gelenkimplantate, Wirbelsäulenimplantate, Radialknochenimplantate, Daumenimplantate und Implantate für die Osteotomie

3. Knochenimplantat nach Anspruch 1, wobei das Knochenimplantat aus einer Gruppe ausgewählt ist, die aus osseointegrierbaren transkutanen Implantaten besteht, die eine Endo-Exo-Prothese für das Bein, eine Endo-Exo-Prothese für die Hand, eine Endo-Exo-Prothese für den Finger und eine Endo-Exo-Prothese für die kosmetische Chirurgie umfassen.

4. Knochenimplantat nach Anspruch 1, wobei das Knochenimplantat aus einer Gruppe von Dentalartikeln ausgewählt ist, die Dentalimplantate, Dentalabutments, Dentalimplantate mit integriertem Abutment, Dentalkronen, Dentalimplantate mit integriertem Abutment und Krone, Dentalbalken, Dentaleinsätze und Dentalstifte umfasst.

## Revendications

1. Implant osseux configuré pour l'ostéo-intégration est caractérisé de sorte que l'implant osseux se compose d'un matériel jade choisi d'un groupe se composant du jade jadéite, du jade néphrite, du jade xonotlite, du jade vésuvianite, du jade pectolite, du jade bowénite et du jade hydro-grand où le matériel de jade est un agrégat du minéral silicate ou un agrégat du minéral de roche avec une structure fibreuse ou finement cristallisée confuse.

2. Implant osseux selon la revendication 1 où l'implant osseux est choisi du groupe se composant d'implants pour le substitut du tissu osseux, des remplisseurs de volume sous forme granulée pour le remplissage des défauts du tissu osseux, des implants osseux cunéiformes, des vis à os, des implants pour la fusion des os, des implants des goujons de fixation ou des goujons de fixation pour les implants, des implants pour la reconstruction des crânes, des implants des articulations de la hanche, des implants des articulations des genoux, des implants des articulations brachiales, des implants des articulations des coudes, des implants des articulations des mains, des implants des articulations des chevilles, des implants des articulations des pieds plats, des implants des articulations des médio-pieds, des implants des articulations des doigts, des implants d'articulation, des implants des colonnes vertébrales, des implants des os radiales, des implants des pouces et des implants pour l'ostéotomie.

3. Implant osseux selon la revendication 1 où l'implant osseux est choisi d'un groupe se composant d'implants ostéo-intégrés transcutanés se composant d'une prothèse articulaire externe pour le pied, d'une prothèse articulaire externe pour la main, d'une prothèse articulaire externe pour les doigts et d'une prothèse articulaire externe pour la chirurgie cosmétique.

4. Implant osseux selon la revendication 1 où l'implant osseux est choisi d'un groupe d'articles dentaires se composant d'implants dentaires, d'abutments dentaires, d'implants dentaires avec abutment intégré, de couronnes dentaires, d'implants dentaires avec abutment intégré et couronne, de poutres dentaires, d'usages dentaires et de pivots dentaires.
